(19) Europäisches Patentamt
European Patent Office
Office européen des brevets

(11) Veröffentlichungsnummer: **0 343 398 A2**

(12) **EUROPÄISCHE PATENTANMELDUNG**

(21) Anmeldenummer: 89107743.0

(22) Anmeldetag: 28.04.89

(51) Int. Cl.4: C07D 215/56 , C07D 471/04 , A61K 31/47 , A61K 31/435 , //(C07D471/04,221:00,221:00)

(30) Priorität: 11.05.88 DE 3816119

(43) Veröffentlichungstag der Anmeldung: 29.11.89 Patentblatt 89/48

(84) Benannte Vertragsstaaten: AT BE CH DE ES FR GB GR IT LI NL SE

(71) Anmelder: BAYER AG

D-5090 Leverkusen 1 Bayerwerk(DE)

(72) Erfinder: Himmler, Thomas, Dr.
Bonhoeffer Strasse 20
D-5000 Köln 80(DE)
Erfinder: Schriewer, Michael, Dr.
Am Theien Siefen 1a
D-5068 Odenthal(DE)
Erfinder: Petersen, Uwe, Dr.
Auf dem Forst 4
D-5090 Leverkusen 1(DE)
Erfinder: Grohe, Klaus, Dr.
Am Wasserturm 10
D-5068 Odenthal(DE)
Erfinder: Haller, Ingo, Dr.
Dornroeschenweg 4
D-5600 Wuppertal 1(DE)
Erfinder: Metzger, Karl Georg, Dr.
Pahlkestrasse 75
D-5600 Wuppertal 1(DE)
Erfinder: Endermann, Rainer, Dr.
In den Birken 152a
D-5600 Wuppertal 1(DE)
Erfinder: Zeiler, Hans-Joachim, Dr.
Elsbeeker Strasse 46
D-5620 Velbert 15(DE)

(54) **7-Substituierte Chinolon- und Naphthyridoncarbonsäure-Derivate.**

(57) Die Erfindung betrifft 7-Aryl- und 7-Hetaryl-substituierte Chinolon- und Naphthyridoncarbonsäure-Derivate der allgemeinen Formel I

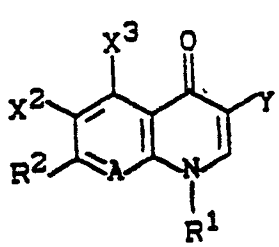

I

in der $R^1$, $R^2$, $X^2$, $X^3$, A und Y die in der Beschreibung angegebene Bedeutung haben, Verfahren zu ihrer Herstellung sowie diese enthaltende antibakterielle Mittel.

EP 0 343 398 A2

# 7-Substituierte Chinolon- und Naphthyridoncarbonsäure-Derivate

Die Erfindung betrifft 7-aryl- und 7-hetaryl-substituierte Chinolon- und Naphthyridoncarbonsäure-Derivate, Verfahren zu ihrer Herstellung sowie diese enthaltende antibakterielle Mittel.

Die gute antibakterielle Wirksamkeit von 7-aryl- bzw. 7-hetaryl-substituierten Chinoloncarbonsäure-Derivaten ist prinzipiell bekannt. So ist z.B. aus der US-PS 3 753 993 die Verbindung 1-Ethyl-1,4-dihydro-4-oxo-7-(4-pyridyl)-3-chinolincarbonsäure (Rosoxacin) bekannt. Die US-PS 4 636 506 beschreibt u.a. die Darstellung von 1-Ethyl-6,8-difluor-7-(4-pyridyl)-1,4-dihydro-4-oxo-3-chinolincarbonsäure. Die EP-PS 184 384 schließlich beschreibt die Synthese von 1-Alkyl-6,(8)-(di)fluor-7-aryl-1,4-dihydro-4-oxo-3-chinolincarbonsäuren.

Der entscheidende C-C-Verknüpfungsschritt für die Arylsubstituenten in der 7-Position im Verfahren der letzt genannten Anmeldung besteht in der durch einen Palladiumkomplex katalysierten Kupplungsreaktion zwischen einem Arylbromid und einem Arylzinkchlorid.

Als Arylbromid kann dabei der 7-Brom-chinoloncarbonsäureester oder ein entsprechend substituiertes Brombenzol, das dann in weiteren Reaktionsschritten zur 7-Arylchinoloncarbonsäure umgesetzt wird, dienen.

Die Nachteile dieses Verfahrens sind in der Beschränkung auf die reaktiven Jod- und Bromarylverbindungen, die Verwendung relativ großer Mengen (6-12 mol-%) eines teuren Palladiumkatalysator wie z.B. Tetrakis-(triphenylphosphin)-palladium (O) und die für die Darstellung der benötigten Arylzinkchloride aus einem Arylbromid, Lithiumalkyl und wasserfreiem Zinkchlorid erforderlichen niedrigen Reaktionstemperaturen von -78° bis -50° C zu sehen.

Es wurde nun gefunden, daß sich überraschenderweise 7-Halogenchinoloncarbonsäureester allgemein, speziell aber auch 7-Chlorchinoloncarbonsäureester, die leichter als die entsprechenden Jod- oder Bromverbindungen herstellbar sind, in Gegenwart eines einfachen und preiswerten Nickelkatalysators mit Arylhalogeniden direkt zu 7-Arylchinoloncarbonsäureestern umsetzen lassen.

Gegenstand der Erfindung ist demnach ein neues Verfahren zur Herstellung von Chinoloncarbonsäure-Derivaten der allgemeinen Formel (I)

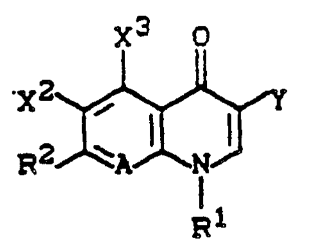

I

in der

$R^1$ für Wasserstoff, gegebenenfalls ein- bis dreifach durch Halogen substituiertes $C_1$-$C_6$-Alkyl bzw. Cycloalkyl, Vinyl, Allyl, Benzyl oder für gegebenenfalls ein- bis dreifach durch $C_1$-$C_3$-Alkyl oder Halogen, insbesondere Fluor substituiertes $C_6$-$C_{10}$-Aryl steht,

$R^2$ für Aryl mit 6 bis 10 Kohlenstoffatomen bzw. Heteroaryl mit 3 bis 9 Kohlenstoffatomen, ein- oder mehrfach substituiert durch $C_1$-$C_4$-Alkyl, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch $C_1$-$C_3$-Alkyl und/oder Fluor substi tuiertes Phenyl, für gegebenenfalls durch $C_1$-$C_3$-Alkyl und/oder Fluor substituiertes Phenoxy, Dialkylamino, Diarylamino, Carboxyalkyl, Alkylsulfinyl, Arylsulfinyl, Alkylsulfonyl, Arylsulfonyl, Formyl, Cyano, Acetoxy oder eine Gruppe

$-\underset{\underset{O}{\|}}{C}-$Alkyl oder $-\underset{\underset{O}{\|}}{C}-$Aryl steht,

$X^2$ für Wasserstoff, $C_1$-$C_3$-Alkyl oder Halogen steht,

$X^3$ für Wasserstoff, $C_1$-$C_3$-Alkyl, Alkoxy mit 1 bis 3 Kohlenstoffatomen oder Halogen steht,

A für Stickstoff oder einen Rest C-$X^4$ steht, worin

$X^4$ Wasserstoff, Halogen oder Cyano sein kann, oder auch gemeinsam mit $R^1$ eine Brücke der Struktur

$-O-CH_2-\underset{|}{C}H-CH_3$, $-S-CH_2-\underset{|}{C}H-CH_3$ oder $-CH_2CH_2\underset{|}{C}HCH_3$

bilden kann,
und
Y eine Nitrilgruppe, eine Estergruppe -$COOR^3$ oder eine Säureamidgruppe -$CONR^4R^5$ darstellt,
wobei
$R^3$ für $C_1$-$C_4$-Alkyl steht und
$R^4$ und $R^5$ gleich oder verschieden sein können und für $C_1$-$C_3$-Alkyl stehen und $R^4$ gegebenenfalls substituiertes Phenyl sein kann,
dadurch gekennzeichnet, daß man 7-Halogenchinoloncarbonsäure-Derivate der Formel II

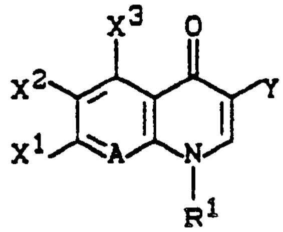

II

in der
$R^1$, $X^2$, $X^3$, A und Y die oben angegebene Bedeutung haben und
$X^1$ für Halogen, bevorzugt Chlor, Brom und Jod, besonders bevorzugt Chlor steht,
in Gegenwart eines Nickelkatalysators mit Arylhalogeniden bzw. Hetarylhalogeniden der Formel III
$R^2$-$X^5$ III
in der
$R^2$ die oben angegebene Bedeutung und
$X^5$ für Halogen außer Fluor, bevorzugt Chlor und Brom steht,
umsetzt.

Bevorzugt ist das Verfahren geeignet zur Herstellung von Chinoloncarbonsäure-Derivaten der Formel I,
in der
$R^1$ für gegebenenfalls durch Halogen, bevorzugt Fluor, substituiertes $C_1$-$C_3$-Alkyl bzw. Cycloalkyl, Vinyl, Alkyl oder für gegebenenfalls ein- bis dreifach durch Halogen, insbesondere Fluor substituiertes Phenyl steht,
$R^2$ für Aryl mit 6 bis 10 Kohlenstoffatomen bzw. Hetaryl mit 3 bis 9 Kohlenstoffatomen, ein- oder mehrfach substituiert durch $C_1$-$C_4$-Alkyl, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch $C_1$-$C_3$-Alkyl und/oder Fluor substituiertes Phenyl, für gegebenenfalls durch $C_1$-$C_3$-Alkyl und/oder Fluor substiutiertes Phenoxy, Dialkylamino, Diarylamino, Carboxyalkyl, Alkylsulfinyl, Arylsulfinyl, Alkylsulfonyl, Arylsulfonyl, Formyl, Cyano, Acetoxy oder eine Gruppe
$-\underset{\underset{O}{\|}}{C}-$Alkyl oder $-\underset{\underset{O}{\|}}{C}-$Aryl steht,
$X^2$ für Wasserstoff oder Halogen, bevorzugt Fluor steht,
$X^3$ Wasserstoff bedeutet,
A für Stickstoff oder einen Rest C-$X^4$ steht,
wobei
$X^4$ für Wasserstoff, Halogen, bevorzugt Fluor und Chlor steht, oder auch gemeinsam mit $R^1$ eine Brücke der Struktur
$-O-CH_2-\underset{|}{C}H-CH_3$, $-S-CH_2-\underset{|}{C}H-CH_3$, $-CH_2CH_2-\underset{|}{C}H-CH_3$
bilden kann, und
Y eine Nitrilgruppe, eine Estergruppe -$COOR^3$ oder eine Säureamidgruppe -$CONR^4R^5$ darstellt,
wobei
$R^3$ für $C_1$-$C_4$-Alkyl steht und
$R^4$ und $R^5$ gleich oder verschieden sein können und für $C_1$-$C_3$-Alkyl stehen und $R^4$ gegebenenfalls substituiertes Phenyl sein kann.

In dieser bevorzugten Ausführungsform ist das Verfahren dadurch gekennzeichnet, daß man 7-Halogenchinoloncarbonsäure-Derivate der Formel (II),

in der
$R^1$, $X^2$, $X^3$, A und Y die oben angegebene Bedeutung haben und
$X^1$ für Halogen, bevorzugt Chlor, Brom besonders bevorzugt Chlor, steht,
in Gegenwart eines Nickelkatalysators mit Arylhalogeniden bzw. Hetarylhalogeniden der Formel III
in der
$X^5$ für Chlor oder Brom steht und
$R^2$ die oben angegebene Bedeutung hat,
umsetzt.

Beispielsweise führt die Umsetzung von 7-Chlor-1-cyclopropyl-6-fluor-1,4-dihydro-4-oxo-3-chinolincarbonsäureethylester mit Chlorbenzol in Gegenwart von Nickel-(II)-chlorid, metallischem Zink und Triphenylphosphin zu 1-Cyclopropyl-6-fluor-1,4-dihydro-4-oxo-7-phenyl-3-chinolincarbonsäureethylester:

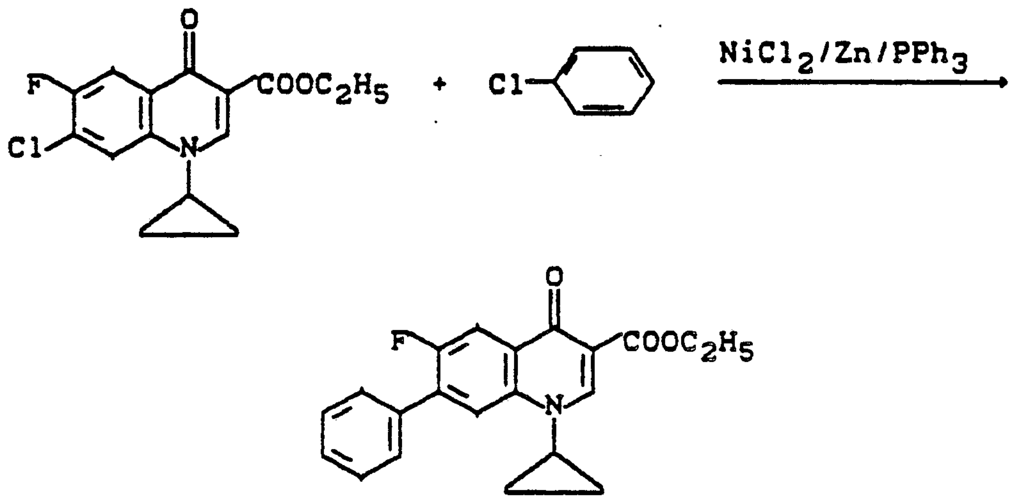


Der in dem erfindungsgemäßen Verfahren zur Anwendung kommende Katalysator enthält eine vorzugsweise wasserfreie Nickelverbindung, einen Liganden aus der Gruppe der Triarylphosphine mit 6 bis 10 C-Atomen in jeder Aryleinheit, einen Promotor aus der Gruppe der Alkali-, Erdalkali-, Mangan- und Zinkhalogenide, -sulfate oder -phosphate und ein Metall wie Zink, Mangan oder Magnesium. Gegebenenfalls kann als weitere Komponente eine polycyclische aromatische Verbindung mit wenigstens 2 Stickstoffatomen in verschiedenen aromatischen Ringen und 10 bis 18 Kohlenstoffatomen zugesetzt werden.

Geeignete wasserfreie Nickelverbindungen sind solche, die durch die genannten Metalle reduziert werden können oder bereits in einer solchen reduzierten Form vorliegen. Ohne Anspruch auf Vollständigkeit seien hier genannt: Nickel(II)-halogenide wie Nickel(II)-chlorid, -bromid und -jodid, Nickel(II)-Komplexe wie Nickelacetylacetonat und Dichloro-bis(triphenylphosphin)nickel(II) und Nickel(O)-Komplexe wie Bis-(cycloocta-1,5-dien)-nickel(O) und Tetrakis(triphenylphosphin)nickel(O), die nach literaturbekannten Verfahren herstellbar sind. Bevorzugt sind die genannten Nickel(II)-halogenide; besonders bevorzugt ist Nickel(II)-chlorid, das sich in bekannter Weise z.B. aus dem Hexahydrat mit Thionylchlorid in wasserfreier Form erhalten läßt.

Der Nickelkatalysator wird in einer Menge von 0,1 bis 25 mol-%, bezogen auf die 7-Halogenchinoloncarbonsäure-Verbindung, bevorzugt 1 bis 20 mol-% und besonders bevorzugt 5 bis 15 mol-% eingesetzt.

Geeignete Triarylphosphine sind z.B. Triphenylphosphin, die Tritolylphoshine und Trinaphthylphosphine. Besonders bevorzugt ist Triphenylphosphin.

Das Triarylphosphin wird in einem Molverhältnis zur Nickelverbindung von 2 bis 100, bevorzugt 4 bis 50 und besonders bevorzugt 5 bis 10 eingesetzt.

Als Promotoren werden Alkali-, Erdalkali-, Zink-, Magnesium- und Manganhalogenide bevorzugt. Besonders bevorzugt sind die Jodide.

Die Menge an Promotor kann 0,1 bis 1000 mol-%, bezogen auf eingesetzten Nickelkatalysator, betragen; bevorzugt sind 0,1 bis 700 mol-% und besonders bevorzugt 10 bis 500 mol-%.

Als bevorzugte polycyclische aromatische Verbindungen mit wenigstens 2 Stickstoffatomen seien 2,2′-Bipyridyl und 1,10-Phenanthrolin genannt, die bevorzugt in Anteilen von 50 bis 500 mol-%, bezogen auf eingesetzten Nickelkatalysator, zugesetzt werden.

Als Metall können beispielsweise Zink, Mangan oder Magnesium verwendet werden. Bevorzugt ist dabei Zink. Das Zink wird beispielsweise als sogenannter Zinkstaub eingesetzt, der gewünschtenfalls vorher durch Waschen mit Eisessig, Wasser und Aceton und anschließendem Trocknen im Vakuum vorbehandelt werden kann.

Das verwendete Metall wird in einer Menge von wenigstens einem halben Moläquivalent, bezogen auf das 7-Halogenchinoloncarbonsäure-Derivat der allgemeinen Formel (II), eingesetzt, bevorzugt jedoch in einem Überschuß von 10 bis 1000 mol-%, besonders bevorzugt 100-500 mol-%, bezogen auf die Verbindung der allgemeinen Formel (II).

Die verwendeten Chinoloncarbonsäurederivate der Formel II sind bekannt.

Als Beispiele seien erwähnt:
7-Chlor-1-cyclopropyl-6-fluor-1,4-dihydro-4-oxo-3-chinolincarbonsäureethylester,
7-Chlor-1-cyclopropyl-6-fluor-1,4-dihydro-4-oxo-3-chinolincarbonsäuremethylester,
7-Chlor-1-cyclopropyl-6-fluor-1,4-dihydro-4-oxo-3-chinolincarbonsäure-n-propylester,
7-Chlor-1-ethyl-6-fluor-1,4-dihydro-4-oxo-3-chinolincarbonsäureethylester,
7-Chlor-6-fluor-1-(p-fluorphenyl)-1,4-dihydro-4-oxo-3-chinolincarbonsäureethylester,
7-Brom-1-cyclopropyl-6-fluor-1,4-dihydro-4-oxo-3-chinolincarbonsäureethylester,
1-Cyclopropyl-6-fluor-1,4-dihydro-7-iodo-4-oxo-3-chinolincarbonsäureethylester,
7-Chlor-1-cyclopropyl-1,4-dihydro-6-methyl-4-oxo-3-chinolincarbonsäureethylester,
7-Chlor-6-fluor-1,4-dihydro-4-oxo-1-vinyl-3-chinolincarbonsäureethylester,
1-Allyl-7-chlor-6-fluor-1,4-dihydro-4-oxo-3-chinolincarbonsäureethylester,
1-Benzyl-7-chlor-6-fluor-1,4-dihydro-4-oxo-3-chinolincarbonsäureethylester,
7-Chlor-8-cyano-1-cyclopropyl-6-fluor-1,4-dihydro-4-oxo-3-chinolincarbonsäureethylester,
7-Chlor-8-cyano-1-ethyl-6-fluor-1,4-dihydro-4-oxo-3-chinolincarbonsäureethylester,
7,8-Dichlor-1-cyclopropyl-6-fluor-1,4-dihydro-4-oxo-3-chinolincarbonsäureethylester,
7-Chlor-1-cyclopropyl-6-fluor-1,4-dihydro-4-oxo-1,8-naphthyridin-3-carbonsäureethylester,
7-Chlor-1-cyclopropyl-6-fluor-1,4-dihydro-4-oxo-3-chinolincarbonitril,
7-Chlor-1-cyclopropyl-6-fluor-1,4-dihydro-4-oxo-3-chinolincarbonsäuredimethylamid.

Geeignete Lösungsmittel für das erfindungsgemäße Verfahren sind beispielsweise aprotische polare Lösungsmittel wie z.B. Dimethylformamid, Dimethylacetamid, N-Methyl-caprolactam, N-Methyl-pyrrolidon, Dimethylsulfoxid, Tetramethylensulfon usw. Die Lösungsmittel können vor Verwendung in bekannter Weise getrocknet und unter einem Schutzgas wie z.B. Stickstoff destilliert werden.

Die Reaktion kann bei Temperaturen zwischen 0 und 250 °C durchgeführt werden, bevorzugt bei 20-200 °C, besonders bevorzugt bei 50 bis 150 °C.

Vorteilhaft wird die Kupplungsreaktion in einer inerten Schutzgasatmosphäre wie z.B. Helium, Argon oder Stickstoff durchgeführt.

Die Reaktion wird normalerweise bei Normaldruck durchgeführt, kann aber auch bei erhöhtem oder vermindertem Druck ablaufen.

Die Reaktionszeit liegt zwischen 0,05 und 24 Stunden, bevorzugt 0,1 und 10 Stunden und besonders bevorzugt 0,5 und 6 Stunden.

Gegenstand der Erfindung sind ebenfalls Verbindungen der Formel IV

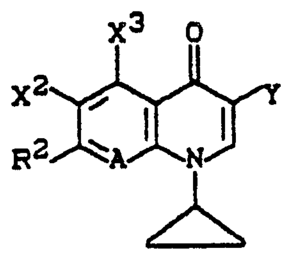

IV

in der
$R^2$ für $C_6$-$C_{10}$-Aryl bzw. $C_3$-$C_9$-Heteroaryl, ein- oder mehrfach substituiert durch $C_1$-$C_4$-Alkyl, Alkoxy mit 1 bis 4 Kohlenstoffatomen, Alkylthio mit 1 bis 4 Kohlenstoffatomen, Halogen, $C_1$-$C_2$-Halogenalkyl mit 1 bis 5 Halogenatomen, gegebenenfalls ein- bis zweifach durch Halogen oder $C_1$-$C_3$-Alkyl substituiertes Phenyl, gegebenenfalls ein- bis zweifach durch Halogen oder $C_1$-$C_3$-Alkyl substituiertes Phenoxy, Nitro, Amino, Alkylamino mit 1 bis 3 Kohlenstoffatomen, Dialkylamino mit 2 bis 6 Kohlenstoffatomen, Aminomethyl, Methylamino, Dimethylaminomethyl, Formylamino, Acetylamino, Acetyloxy, Formyl, Acetyl, Carboxyalkyl mit 1 bis 3 Kohlenstoffatomen im Säurerest, Alkoxycarbamoyl mit 1 bis 3 Kohlenstoffatomen in Alkoxyteil,

Cyano, Alkylsulfinyl mit 1 bis 3 Kohlenstoffatomen, Alkylsulfonyl mit 1 bis 3 Kohlenstoffatomen, Arylsulfinyl, Arylsulfonyl, Sulfamoyl, Alkylsulfamoyl mit 1-3 Kohlenstoffatomen oder Dialkylsulfamoyl mit 2 bis 6 Kohlenstoffatomen steht,
$X^2$ für Wasserstoff, $C_1$-$C_3$-Alkyl, Nitro oder Halogen steht,
$X^3$ Wasserstoff, $C_1$-$C_3$-Alkyl, Nitro, $C_1$-$C_3$-Alkoxy oder Halogen bedeutet,
A für Stickstoff oder einen Rest C-$X^4$ steht, worin $X^4$ für Wasserstoff, $C_1$-$C_3$-Alkyl, Halogen, Nitro oder Cyano steht und
Y eine Nitrilgruppe, eine Gruppe $COOR^3$ oder eine Säureamidgruppe $CONR^4R^5$ darstellt, wobei $R^3$ für Wasserstoff oder für $C_1$-$C_4$-Alkyl steht und $R^4$ und $R^5$ gleich oder verschieden sein können und für $C_1$-$C_3$-Alkyl stehen und $R^4$ gegebenenfalls substituiertes Phenyl sein kann.

Bevorzugt sind Verbindungen der Formel IV in der
$R^2$ für folgende Aryl- bzw. Hetarylreste

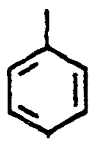 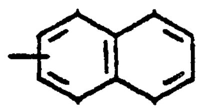 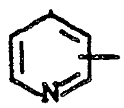 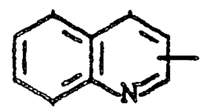

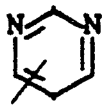 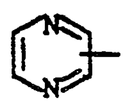 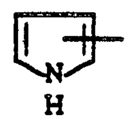 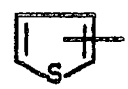

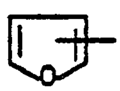 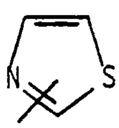 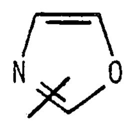 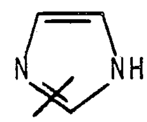 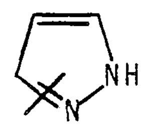 ,

ein oder mehrfach substituiert durch $C_1$-$C_4$-Alkyl, Alkoxy mit 1 bis 3 Kohlenstoffatomen, Alkylmercapto mit 1 bis 3 Kohlenstoffatomen, Halogen, gegebenenfalls ein- bis zweifach durch Halogen, bevorzugt Fluor, oder $C_1$-$C_3$-Alkyl substituiertes Phenyl, Nitro, Amino, Alkylamino mit 1 bis 3 Kohlenstoffatomen, Dialkylamino mit 2 bis 6 Kohlenstoffatomen, Aminomethyl, Methylaminomethyl, Dimethylaminomethyl, Formylamino, Acetylamino, Acetyloxy, Alkoxycarbonyl mit 1 bis 3 Kohlenstoffatomen im Alkoxyteil, Cyano, Sulfamoyl, Alkylsulfamoyl mit 1 bis 3 Kohlenstoffatomen oder Dialkylsulfamoyl mit 2 bis 6 Kohlenstoffatomen steht,
$X^2$ für Wasserstoff oder Halogen steht,
$X^3$ Wasserstoff oder Halogen bedeutet,
A Stickstoff oder einen Rest C-$X^4$ darstellt, worin $X^4$ für Wasserstoff, Halogen oder Cyano steht und
Y eine Nitrilgruppe oder eine Gruppe $COOR^3$ bedeutet, wobei $R^3$ für Wasserstoff oder für $C_1$ bis $C_4$-Alkyl steht.

Besonders bevorzugt sind Verbindungen der Formel IV in der
$R^2$ für folgende Aryl- bzw. Hetarylreste

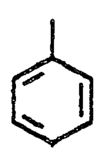 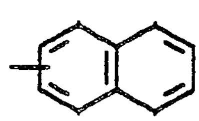 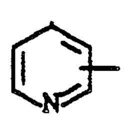 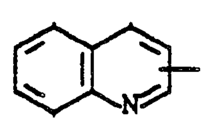

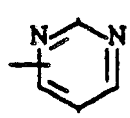 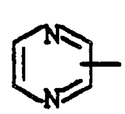 ,

ein oder mehrfach substituiert durch $C_1$-$C_3$-Alkyl, Alkoxy mit 1 bis 3 Kohlenstoffatomen, Methylmercapto, Halogen, gegebenenfalls ein bis zweifach durch Halogen, insbesondere Fluor, oder Methyl substituiertes Phenyl, Nitro, Amino, Alkylamino mit 1 bis 3 Kohlenstoffatomen, Dialkylamino mit 2 bis 4 Kohlenstoffatomen, Aminomethyl, Methylaminomethyl, Dimethylaminomethyl, Sulfamoyl, Alkylsulfamoyl mit 1-3 Kohlenstoffatomen oder Dialkylsulfamoyl mit 2-4 Kohlenstoffatomen steht,

$X^2$ für Fluor steht,

$X^3$ Wasserstoff bedeutet,

A Stickstoff oder einen Rest C-$X^4$ darstellt, worin $X^4$ für Wasserstoff, Fluor oder Chlor steht,

und

Y eine Gruppe $COOR^3$ bedeutet, wobei $R^3$ für Wasserstoff, Methyl oder Ethyl steht.

Die erfindungsgemäßen Verbindungen der Formel IV sind größtenteils nach dem zuvor beschriebenen Verfahren erhältlich.

Bei der Herstellung der erfindungsgemäßen Verbindungen kann es jedoch auch notwendig sein, manche Gruppen erst nach dem entscheidenden C-C-Verknüpfungsschritt einzuführen, weil deren Anwesenheit in Verbindungen der Formel II oder Verbindungen der Formel III den C-C-Verknüpfungsschritt unterbinden würde.

Derartige Gruppen wie Amino, Nitro etc. können nach bekannten Methoden der präparativen organischen Chemie eingeführt werden.

Dies sei beispielhaft durch folgendes Formelschema erläutert:

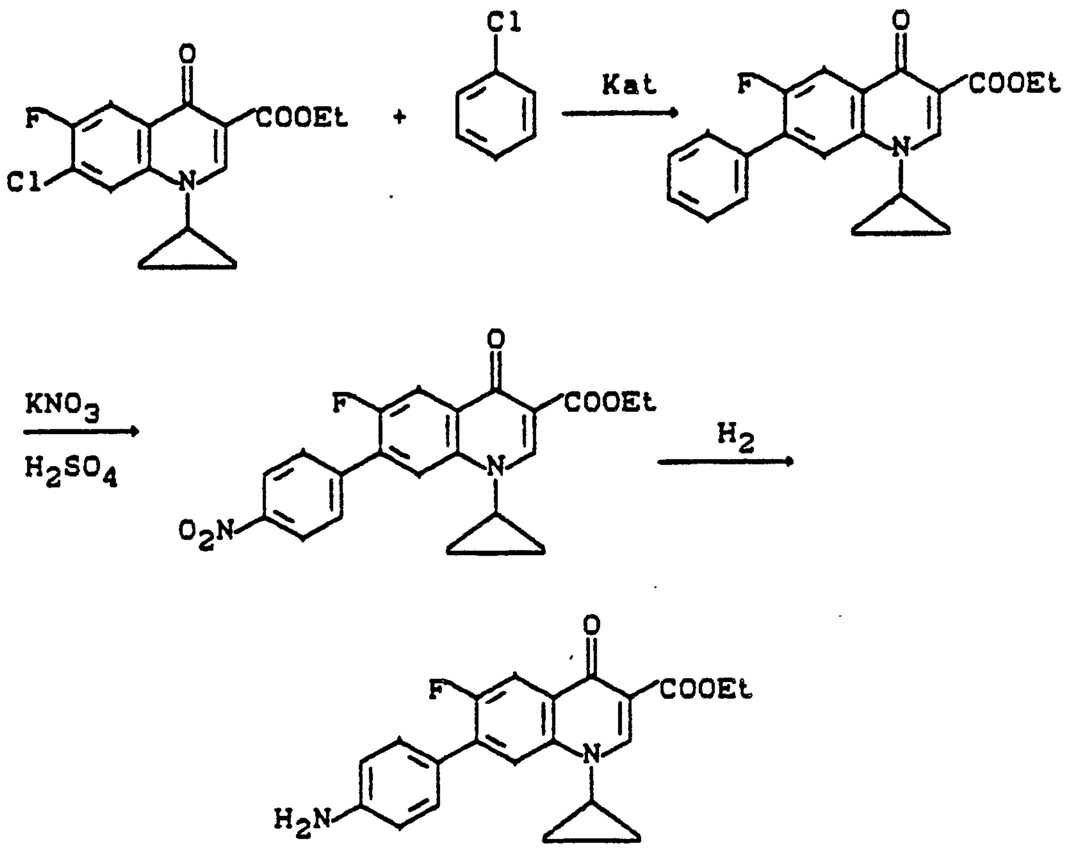


Ebenso kann es notwendig sein, reaktivere Zentren an den Verbindungen II bzw. III mit Schutzgruppen zu versehen, um den C-C-Verknüpfungsschritt durchführen zu können. Durch die in der organischen Chemie bekannten Verfahren zur Abspaltung dieser Schutzgruppen können die erfindungsgemäßen Verbindungen der Formel IV erhalten werden.

Außer den in den Beispielen aufgeführten Verbindungen sind als weitere Wirkstoffe der Erfindung die in Tabelle 1 aufgeführten Verbindungen zu nennen.

Tabelle 1 Verbindungen der Formel IV

| $X^2$ | $X^3$ | A | $R^2$ | Y |
|---|---|---|---|---|
| F | H | CH | 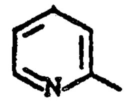 | COOH |
| F | F | CH | 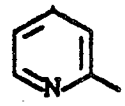 | COOH |
| F | H | CF | 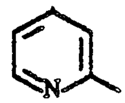 | COOH |
| F | H | CH | 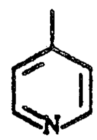 | COOH |
| F | H | CH | 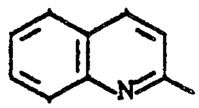 | COOH |
| F | H | CH | 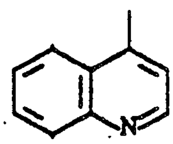 | COOH |
| F | H | N | 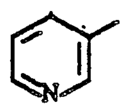 | COOH |
| F | H | N | 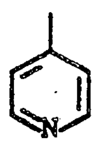 | COOH |
| F | H | N | 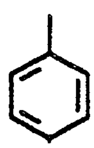 | COOH |
| F | H | N | 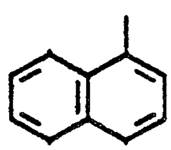 | COOH |

Tabelle 1 (Fortsetzung)

| $X^2$ | $X^3$ | A | $R^2$ | Y |
|---|---|---|---|---|
| F | H | N | 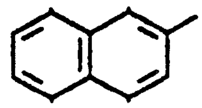 | COOH |
| F | H | CH | 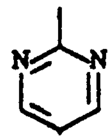 | COOH |
| F | H | CH | 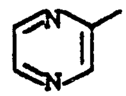 | COOH |
| F | H | CH | 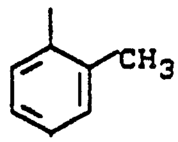 | COOH |
| F | H | CH | 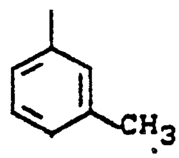 | COOH |
| F | H | CH | 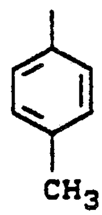 | COOH |
| F | H | N | 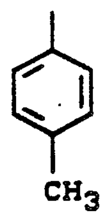 | COOH |
| F | F | CH | 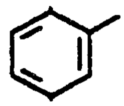 | COOH |

<u>Tabelle 1</u> (Fortsetzung)

| $X^2$ | $X^3$ | A | $R^2$ | Y |
|---|---|---|---|---|
| F | F | CH | 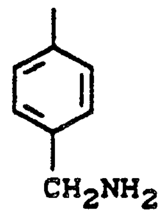 | COOH |
| F | H | CH | 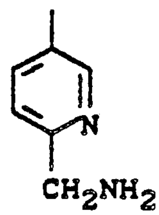 | COOH |
| F | H | CH | 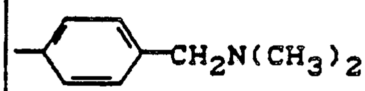 | COOH |
| F | H | N | 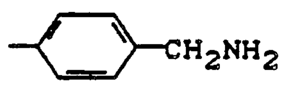 | COOH |
| F | H | CH | 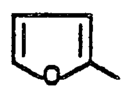 | COOH |
| F | H | CH | 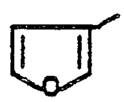 | COOH |
| F | H | CH | 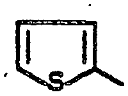 | COOH |
| F | H | CH | 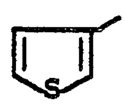 | COOH |

Tabelle 1 (Fortsetzung)

| $X^2$ | $X^3$ | A | $R^2$ | Y |
|---|---|---|---|---|
| F | H | CH | 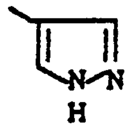 | COOH |
| F | H | CH | 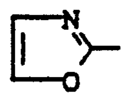 | COOH |
| F | H | CH | 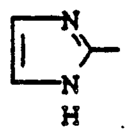 | COOH |
| F | H | CH | 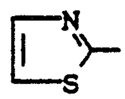 | COOH |
| F | $CH_3$ | CH | 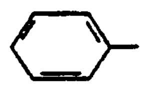 | COOH |
| F | $CH_3$ | CH | 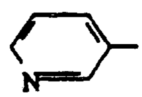 | COOH |
| F | $CH_3$ | CH | 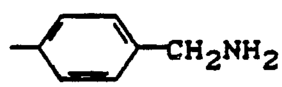 | COOH |
| F | H | CH | 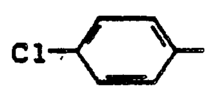 | COOH |
| F | H | CH | 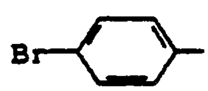 | COOH |

T a b e l l e 1 (Fortsetzung)

| $X^2$ | $X^3$ | A | $R^2$ | Y |
|---|---|---|---|---|
| F | H | CH | 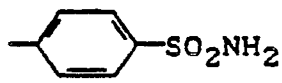 | COOH |
| F | H | CH | 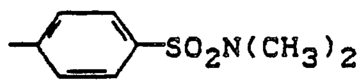 | COOH |
| F | H | CH | 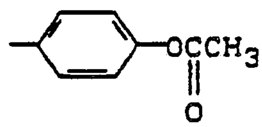 | COOH |
| F | H | CH | 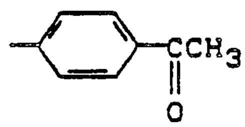 | COOH |
| F | H | CH | 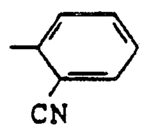 | COOH |
| F | H | CH | 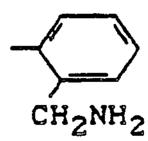 | COOH |
| F | H | CH | 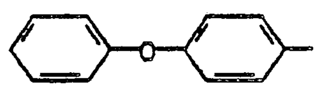 | COOH |
| F | H | CH | 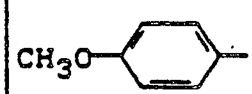 | COOH |
| F | H | CH | 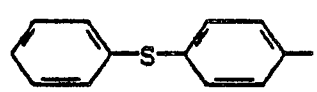 | COOH |

Tabelle 1 (Fortsetzung)

| $X^2$ | $X^3$ | A | $R^2$ | Y |
|---|---|---|---|---|
| F | H | N | 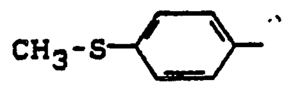 | COOH |
| F | H | CH | 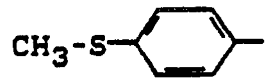 | COOH |
| F | H | N | 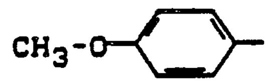 | COOH |
| F | H | CH | 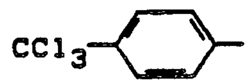 | COOH |
| F | H | CH | 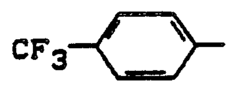 | COOH |
| F | H | CH | 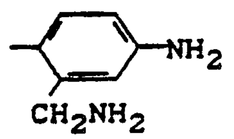 | COOH |
| F | H | CH | 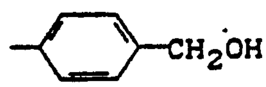 | COOH |
| F | H | CH | 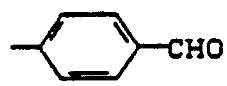 | COOH |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Haemophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung

Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, post-operative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Mastitis-Metritis-Agalaktie-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll-und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Ad sorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt

sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formu lierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte einiger der erfindungsgemäßen Verbindungen im Vergleich zu Ciprofloxacin angegeben.

MHK-Werte (µg/ml) von Verbindungen der Formel

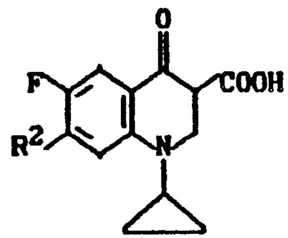

| $R^2$ | Bei-spiel | E.coli Neumann | Staph. FK422 | Staph. 1756 | Staph. 133 | Enteroc. 27101 | Enterco. 9790 | Pseudo-monas Walter | Pseudo-monas Ellsworth |
|---|---|---|---|---|---|---|---|---|---|
| 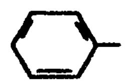 | 1 | 0,062 | ≤0,015 | 0,062 | 0,062 | 0,062 | 0,125 | 0,5 | 0,25 |
| 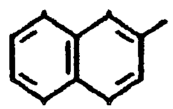 | 14 | 0,062 | ≤0,015 | ≤0,015 | ≤0,015 | ≤0,015 | 0,062 | 1 | 0,25 |
| 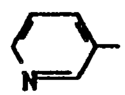 | 16 | ≤0,015 | 0,031 | 0,031 | 0,031 | 0,125 | 0,125 | 0,5 | 0,125 |
| 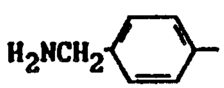 | 20 | ≤0,015 | 0,031 | 0,031 | 0,031 | 0,062 | 0,062 | 0,062 | 0,062 |
| (Ciprofloxacin) | | ≤0,015 | 0,25 | 0,25 | 0,25 | 0,25 | 0,25 | 0,25 | 0,25 |

Beispiel 1

1-Cyclopropyl-6-fluor-1,4-dihydro-4-oxo-7-phenyl-3-chinolincarbonsäure-ethylester

In einer Stickstoffatmosphäre werden 6,7 g Zinkpulver, 6,7 g Triphenylphosphin, 1 g Natriumjodid, 0,435 g Nickel(II)-chlorid und 50 ml Dimethylformamid vorgelegt. Nach einer halben Stunde bei 50-60° C liegt eine tief rot-braune Suspension vor. In diese wird eine warme Lösung von 7,75 g 7-Chlor-1-cyclopropyl-6-fluor-1,4-dihydro-4-oxo-3-chinolincarbonsäure-ethylester und 5,65 g Chlorbenzol in 50 ml Dimethylformamid gegeben. Nach einer halben Stunde bei 80° C unter Stickstoff wird das Reaktionsgemisch heiß filtriert. Nach Erkalten des Filtrats wird erneut filtriert und der Rückstand mit Dimethylformamid und Methylenchlorid/n-Hexan gewaschen. Nach Trocknen erhält man 4 g 1-Cyclopropyl-6-fluor 1,4-dihydro-4-oxo-7-phenyl-3-chinolincarbonsäure-ethylester (45,5 %) der Theorie); Schmelzpunkt: 262-263° C.
$^1$H-NMR (200 MHz, $CDCl_3$): 1,1-1,5 (m; 7H), 3,55 (m; 1H), 4,4 (q; 2H), 7,45-7,7 (m; 5H), 7.95 (d, J ~ 6 Hz; 1H), 8,09 (d, J ~ 11 Hz; 1H), 8,56 (s; 1H) ppm.

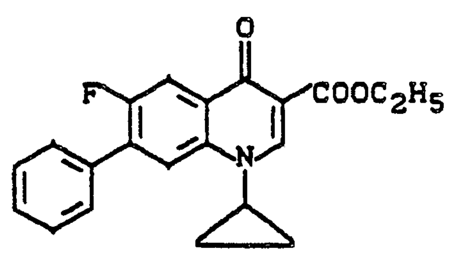

Beispiel 2

1-Cylopropyl-6-fluor-1,4-dihydro-4-oxo-7-phenyl-3-chinolincarbonsäure

3,5 g 1-Cyclopropyl-6-fluor-1,4-dihydro-4-oxo-7-phenyl-3-chinolincarbonsäure-ethylester werden in 60 ml Eisessig, 20 ml Wasser und 2 ml konzentrierter Schwefelsäure 4 Stunden unter Rückfluß erhitzt. Nach Erkalten der Reaktionsmischung wird filtriert, der Rückstand mit Eisessig und Wasser gewaschen und getrocknet. Man erhält 2,86 g 1-Cyclopropyl-6-fluor-1,4-dihydro-4-oxo-7-phenyl-3-chinolincarbonsäure (88,8 % der Theorie); Schmelzpunkt: 268° C.

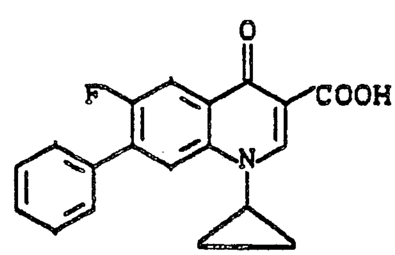

In entsprechender Weise wurden die in der Tabelle 2 aufgeführten Verbindungen erhalten.

Tabelle 2

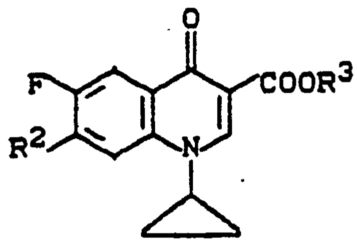

| Beispiel | $R^2$ | $R^3$ | Schmelzpunkt [°C] |
|---|---|---|---|
| 3 | 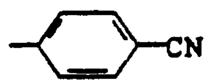 | $C_2H_5$ | 245 |
| 4 | " | H | ›290 |
| 5 | 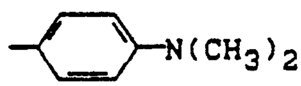 | $C_2H_5$ | 262-267 |
| 6 | " | H | 257-258 |
| 7 | 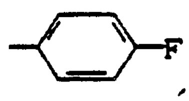 | $C_2H_5$ | 206-208 |
| 8 | " | H | ›295 |
| 9 | 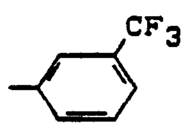 | $C_2H_5$ | - |
| 10 | " | H | 236-240 |
| 11 | 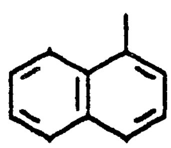 | $C_2H_5$ | - |
| 12 | " | H | 244-245 |

## Tabelle 2

| Beispiel | $R^2$ | $R^3$ | Schmelzpunkt [°C] |
|---|---|---|---|
| 13 | 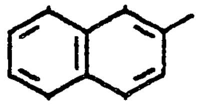 | $C_2H_5$ | - |
| 14 | " | H | 300 (Zers.) |
| 15 | 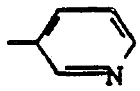 | $C_2H_5$ | 249-253 |
| 16 | " | H | 260-261 |
| 17 | 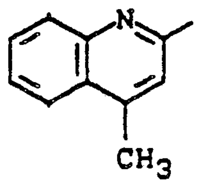 | $C_2H_5$ | - |
| 18 | " | H | 280 (Zers.) |

Beispiel 19

7-(4-Aminomethyl-phenyl)-1-cyclopropyl-6-fluor-1,4-dihydro-4-oxo-3-chinolincarbonsäure-ethylester

2 g 7-(4-Cyanophenyl)-1-cyclopropyl-6-fluor-1,4-dihydro-4-oxo-3-chinolincarbonsäure-ethylester werden in 130 ml Tetrahydrofuran in Gegenwart von 20 ml Ammoniak und 2 g Raney-Kobalt bei 140-150 bar Wasserstoffdruck hydriert.

Das Reaktionsgemisch wird filtriert, das Lösungsmittel abgezogen und der Rückstand in Chloroform gelöst. Diese Lösung wird mit verdünnter wäßriger Salzsäure ausgeschüttelt. Die wäßrige Phase wird verdünnt, mit wäßriger Natronlauge alkalisch gestellt und mit Chloroform ausgeschüttelt. Nach Trocknen der organischen Phase und Abziehen des Lösungsmittels erhält man 1 g 7-(4-Aminomethyl-phenyl)-1-cyclopropyl-6-fluor-1,4-dihydro-4-oxo-3-chinolincarbonsäure-ethylester (49,5 % der Theorie).

$^1$H-NMR (200 MHz, $CDCl_3$): 1,1-1,5 (m; 7H), 1,7 (s, br; 2H), 3,5 (m; 1H), 3,95 (s; 2H), 4,39 (q; 2H), 7,55-7,65 (m; 4H), 7,95 (d,J ~ 6 Hz; 1H), 8,15 (d, J ~ 11 Hz; 1H), 8,85 (s; 1H) ppm.

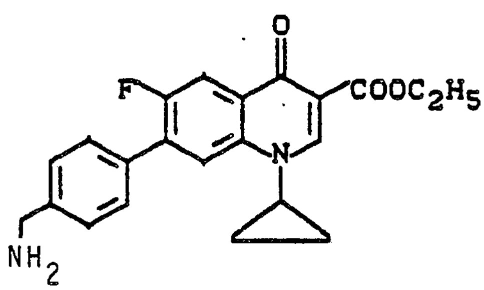

Beispiel 20

7-(4-Aminomethylphenyl)-1-cyclopropyl-6-fluor-1,4-dihydro-4-oxo-3-chinolincarbonsäure

0,5 g 7-(4-Aminomethylphenyl)-1-cyclopropyl-6-fluor-1,4-dihydro-4-oxo-3-chinolincarbonsäure-ethylester werden in 2 ml Eisessig, 0,5 ml Wasser und 0,2 ml konzentrierter Schwefelsäure 4 Stunden unter Rückfluß gekocht. Nach Erkalten wird das Reaktionsgemisch filtriert, der Rück stand mit Eisessig und Wasser gewaschen und getrocknet. Man erhält so 0,22 g 7-(4-Aminomethylphenyl)-1-cyclopropyl-6-fluor-1,4-dihydro-4-oxo-3-chinolincarbonsäure (27,5 % der Theorie). Schmelzpunkt: 285° C (Zers.).

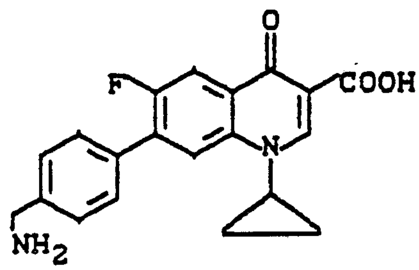

Beispiel 21

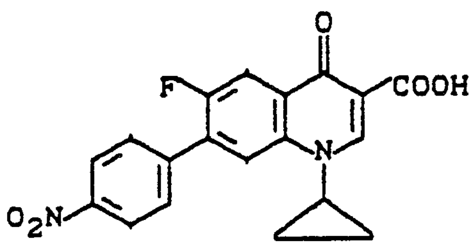

1,0 g 1-Cyclopropyl-6-fluor-1,4-dihydro-4-oxo-7-phenyl-3-chinolincarbonsäure werden in 10 ml Schwefelsäure vorgelegt. Unter Eiskühlung werden 0,47 g Kaliumnitrat portionsweise zugegeben. Danach läßt man langsam auf Raumtemperatur kommen und erwärmt anschließend 3 Stunden auf 50°. Nach Versetzen mit Eis wird der gebildete Feststoff isoliert, gewaschen, getrocknet und aus Dimethylformamid umkristallisiert. Ausbeute: 0,3 g 1-Cyclopropyl-6-fluor-1,4-dihydro-7-(4-nitro-phenyl)-4-oxo-3-chinolincarbonsäure. Schmelzpunkt 304-5°.

Beispiel 22

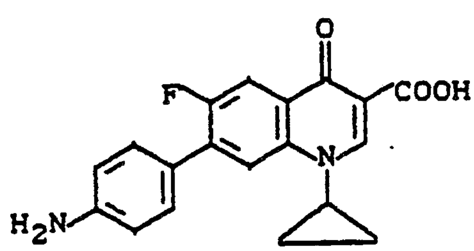

1 g 1-Cyclopropyl-6-fluor-1,4-dihydro-7-(4-nitro-phenyl)-4-oxo-3-chinolincarbonsäure werden in 50 ml Dimethylformamid unter Zugabe von 1 g Raney-Nickel hydriert. Nach Beendigung der Wasserstoffaufnahme wird vom Katalysator filtriert. Das Filtrat wird eingeengt und der Rückstand mit verdünnter Salzsäure verrührt. Der gebildete Feststoff wird isoliert, mit Ethanol gewaschen und getrocknet. Ausbeute 0,8 g 7-(4-Amino-phenyl)-1-cyclopropyl-6-fluor-1,4-dihydro-4-oxo-3-chinolincarbonsäure als Hydrochlorid. Schmelzpunkt >270°.

**Ansprüche**

1. Verfahren zur Herstellung von Chinoloncarbonsäure-Derivaten der allgemeinen Formel (I),

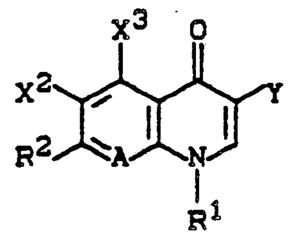

I

in der
$R^1$ für Wasserstoff, gegebenenfalls ein- bis dreifach durch Halogen substituiertes $C_1$-$C_6$-Alkyl bzw. Cycloalkyl, Vinyl, Allyl, Benzyl oder für gegebenenfalls ein- bis dreifach durch $C_1$-$C_3$-Alkyl oder Halogen, insbesondere Fluor substituiertes $C_6$-$C_{10}$-Aryl steht,
$R^2$ für Aryl mit 6 bis 10 Kohlenstoffatomen bzw. Heteroaryl mit 3 bis 9 Kohlenstoffatomen, ein-oder mehrfach substituiert durch $C_1$-$C_4$-Alkyl, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch $C_1$-$C_3$-Alkyl und/oder Fluor substituiertes Phenyl, für gegebenenfalls durch $C_1$-$C_3$-Alkyl und/oder Fluor substituiertes Phenoxy, Dialkylamino, Diarylamino, Carboxyalkyl, Alkylsulfinyl, Arylsulfinyl, Alkylsulfonyl, Arylsulfonyl, Formyl, Cyano, Acetoxy, oder eine Gruppe
$-\underset{\underset{O}{\|}}{C}-$Alkyl oder $-\underset{\underset{O}{\|}}{C}-$Aryl steht,
$X^2$ für Wasserstoff, $C_1$-$C_3$-Alkyl oder Halogen steht,
$X^3$ für Wasserstoff, $C_1$-$C_3$-Alkyl, Alkoxy mit 1 bis 3 Kohlenstoffatomen oder Halogen steht,
A für Stickstoff oder einen Rest C-$X^4$ steht, worin
$X^4$ Wasserstoff, Halogen oder Cyano sein kann, oder auch gemeinsam mit $R^1$ eine Brücke der Struktur
$-O-CH_2-\underset{|}{C}H-CH_3$, $-S-CH_2-\underset{|}{C}H-CH_3$ oder $-CH_2CH_2\underset{|}{C}HCH_3$ .
bilden kann,
und
Y eine Nitrilgruppe, eine Estergruppe $-COOR^3$ oder eine Säureamidgruppe $-CONR^4R^5$ darstellt,
wobei
$R^3$ für $C_1$-$C_4$-Alkyl steht und
$R^4$ und $R^5$ gleich oder verschieden sein können und für $C_1$-$C_3$-Alkyl stehen und $R^4$ gegebenenfalls substituiertes Phenyl sein kann,
dadurch gekennzeichnet, daß man 7-Halogenchinoloncarbonsäure-Derivate der Formel II

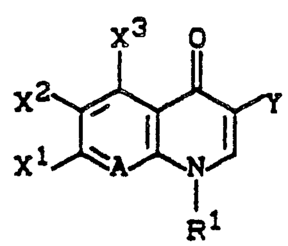

II

in der
$R^1$, $X^2$, $X^3$, A und Y die oben angegebene Bedeutung haben und
$X^1$ für Halogen, bevorzugt Chlor, Brom und Jod, besonders bevorzugt Chlor steht,
in Gegenwart eines Nickelkatalysators mit Arylhalogeniden bzw. Hetarylhalogeniden der Formel III
$R^2$-$X^5$ III
in der
$R^2$ die oben angegebene Bedeutung hat und
$X^5$ für Halogen außer Fluor, bevorzugt Chlor und Brom steht,
umsetzt.

2. Verfahren nach Anspruch 1, dadurch gekennzeichnet, daß der Katalysator eine wasserfreie Nickelverbindung ist.

3. Verfahren nach Anspruch 1 und 2, dadurch gekennzeichnet, daß der Katalysator eine wasserfreie Nickelverbindung und einen Liganden aus der Gruppe der Triarylphosphine mit 6 bis 10 C-Atomen in jeder Aryleinheit enthält.

4. Verfahren nach den Ansprüchen 1 bis 3, dadurch gekennzeichnet, daß der Katalysator einen Promotor aus der Gruppe der Alkali-, Erdalkali-, Mangan- und Zinkhalogenide, -sulfate oder -phosphate, ein Metall wie Zink, Mangan oder Magnesium und gegebenenfalls eine polycyclische aromatische Verbin dung mit mindestens 2 Stickstoffatomen in verschiedenen aromatischen Ringen mit 10 bis 18 C-Atomen enthält.

5. Verbindungen der Formel IV

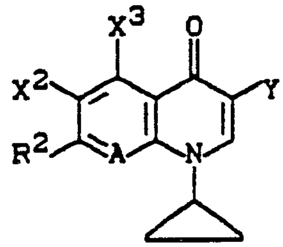

IV

in der
$R^2$ für $C_6$-$C_{10}$-Aryl bzw. $C_3$-$C_9$-Heteroaryl ein-oder mehrfach substituiert durch $C_1$-$C_4$-Alkyl, Alkoxy mit 1 bis 4 Kohlenstoffatomen, Alkylthio mit 1 bis 4 Kohlenstoffatomen, Halogen, $C_1$-$C_2$-Halogenalkyl mit 1 bis 5 Halogenatomen, gegebenenfalls ein- bis zweifach durch Halogen oder $C_1$-$C_3$-Alkyl substituiertes Phenyl, gegebenenfalls ein- bis zweifach durch Halogen oder $C_1$-$C_3$-Alkyl substituiertes Phenoxy, Nitro, Amino, Alkylamino mit 1 bis 3 Kohlenstoffatomen, Dialkylamino mit 2 bis 6 Kohlenstoffatomen, Aminomethyl, Methylamino, Dimethylaminomethyl, Formylamino, Acetylamino, Acetyloxy, Formyl, Acetyl, Carboxyalkyl mit 1 bis 3 Kohlenstoffatomen im Säurerest, Alkoxycarbamoyl mit 1 bis 3 Kohlenstoffatomen in Alkoxyteil, Cyano, Alkylsulfinyl mit 1 bis 3 Kohlenstoffatomen, Alkylsulfonyl mit 1 bis 3 Kohlenstoffatomen, Arylsulfinyl, Arylsulfonyl, Sulfamoyl, Alkylsulfamoyl mit 1-3 Kohlenstoffatomen oder Dialkylsulfamoyl mit 2 bis 6 Kohlenstoffatomen stehen,
$X^2$ für Wasserstoff, $C_1$-$C_3$-Alkyl, Nitro oder Halogen steht,
$X^3$ Wasserstoff, $C_1$-$C_3$-Alkyl, Nitro, $C_1$-$C_3$-Alkoxy oder Halogen bedeutet,
A für Stickstoff oder einen Rest C-$X^4$ steht, worin $X^4$ für Wasserstoff, $C_1$-$C_3$-Alkyl, Halogen, Nitro oder Cyano steht und
Y eine Nitrilgruppe, eine Gruppe $COOR^3$ oder eine Säureamidgruppe $CONR^4R^5$ darstellt, wobei $R^3$ für Wasserstoff oder für $C_1$-$C_4$-Alkyl steht und $R^4$ und $R^5$ gleich oder verschieden sein können und für $C_1$-$C_3$-Alkyl stehen und $R^4$ gegebenenfalls substituiertes Phenyl sein kann.

6. Verbindungen der Formel IV nach Anspruch 5,
in der
$R^2$ für folgende Aryl- bzw. Hetarylreste

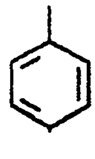 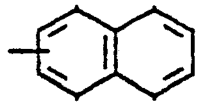 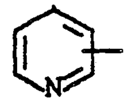 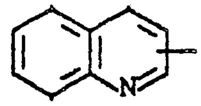

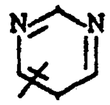 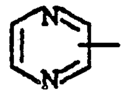 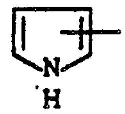 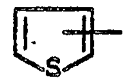

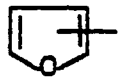 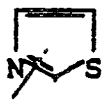 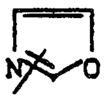 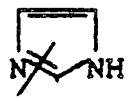 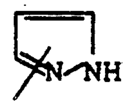 ,

ein oder mehrfach substituiert durch $C_1$-$C_3$-Alkyl, Alkoxy mit 1 bis 3 Kohlenstoffatomen, Akylmercapto mit 1 bis 3 Kohlenstoffatomen, Halogen, gegebenenfalls ein- bis zweifach durch Halogen, bevorzugt Fluor, oder $C_1$-$C_3$-Alkyl substituiertes Phenyl, Nitro, Amino, Alkylamino mit 1 bis 3 Kohlenstoffatomen, Dialkylamino mit 2 bis 6 Kohlenstoffatomen, Aminomethyl, Methylaminomethyl, Dimethylaminomethyl, Formylamino, Acetylamino, Acetyloxy, Alkoxycarbonyl mit 1 bis 3 Kohlenstoffatomen im Alkoxyteil, Cyano, Sulfamoyl, Alkylsulfamoyl mit 1 bis 3 Kohlenstoffatomen oder Dialkylsulfamoyl mit 2 bis 6 Kohlenstoffatomen steht,
$X^2$ für Wasserstoff oder Halogen steht,
$X^3$ Wasserstoff oder Halogen bedeutet,
A Stickstoff oder einen Rest C-$X^4$ darstellt, worin $X^4$ für Wasserstoff, Halogen oder Cyano steht
und
Y Wasserstoff, eine Nitrilgruppe oder eine Estergruppe $COOR^3$ bedeutet, wobei $R^3$ für $C_1$ bis $C_4$-Alkyl steht.

7. Verbindungen der Formel IV nach Anspruch 5,
in der
$R^2$ für die Aryl- bzw. Hetarylreste

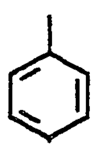 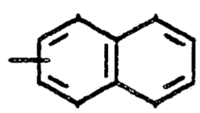 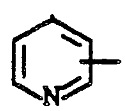 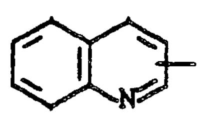

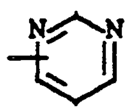 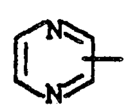 ,

ein oder mehrfach substituiert durch $C_1$-$C_3$-Alkyl, Alkoxy mit 1 bis 3 Kohlenstoffatomen, Methylmercapto, Halogen, gegebenenfalls ein bis zweifach durch Halogen, insbesondere Fluor, oder Methyl substituiertes Phenyl, Nitro, Amino, Alkylamino mit 1 bis 3 Kohlenstoffatomen, Dialkylamino mit 2 bis 4 Kohlenstoffatomen, Aminomethyl, Methylaminomethyl, Dimethylaminomethyl, Sulfamoyl, Alkylsulfamoyl mit 1-3 Kohlenstoffatomen oder Dialkylsulfamoyl mit 2-4 Kohlenstoffatomen steht,
$X^2$ für Fluor steht,
$X^3$ Wasserstoff bedeutet,
A Stickstoff oder einen Rest C-$X^4$ darstellt, worin $X^4$ für Wasserstoff, Fluor oder Chlor steht
und
Y eine Gruppe $COOR^3$ bedeutet, wobei $R^3$ für Wasserstoff, Methyl oder Ethyl steht.

8. Arzneimittel, enthaltend Verbindungen der Formel IV gemäß Anspruch 5.

9. Verbindungen der Formel IV gemäß Anspruch 5 zur Anwendung in einem Verfahren zur therapeutischen Behandlung des menschlichen oder tierischen Körpers.

10. Verwendung von Verbindungen der Formel IV gemäß Anspruch 5 zur Herstellung von Arzneimitteln.